# NOUVEAU FASCICULE DE BREVET EUROPEEN

(11) **EP 0 394 152 B2**
(45) Date de publication et mention de la décision concernant l'opposition: **22.03.2006**
(45) Mention de la délivrance du brevet: 22.06.1994
(21) Numéro de dépôt: 90420194.4
(22) Date de dépôt: 18.04.1990
(51) Int. Cl.: A61L 27/00

(54) **Matériau et procédé de restauration d'un défaut osseux par comblement par du tissu osseux**
Material und Verfahren zur Restauration eines Knochendefekts mittels Knochengewebe
Material and process for rectifying bone defects with bone tissue

(30) Priorité: 20.04.1989 FR 8905504
(43) Date de publication de la demande: 24.10.1990
(73) Titulaire: IMPLANT INNOVATIONS, INC., Palm Beach Gardens, Florida 33410 (US)
(72) Inventeur: Ducheyne, Paul, Bryn Nawr, PA 9010 (US); Schepers, Evert, B-3000 Leuven (BE); Kempeneers, Raymond, B-2400 Mol (BE); De Clercq, Marcel, Rotselaar (BE)
(74) Mandataire: Stebbing, Peter John Hunter

(56) Documents cités:
- EP-A- 0 145 210
- EP-A- 0 206 726
- EP-A- 0 206 726
- EP-A- 0 263 489
- FR-A- 2 606 282
- US-A- 4 159 358
- Journal of Oral Rehabilitation 1997, 24 p171-181
- Journal of Biomed.Mater.Res. 1998,p527-533

## Description

L'invention concerne un matériau sous forme de granulé pour procéder au comblement rapide et complet d'un défaut osseux par formation de tissus osseux.

On sait que des produits comme l'hydroxylapatite dense ou poreux, le phosphate tricalcique ou les bioverres peuvent être utilisés sous forme de granulés comme matériau de remplissage pour des lésions osseuses telles que poches parodontales, sites d'extraction, lésions cystiques, défaut osseux...

Dans le cas du remplissage de cavités osseuses, le rôle de ces granulés (notamment d'hydroxylapatite) est d'agir comme matrice en vue d'une restauration par croissance osseuse (c.à.d. prolifération normale de cellules osseuses). Il a ainsi été démontré qu'il se produit une ostéoconduction (c'est-à-dire une croissance de tissus osseux le long de la surface des granulés implantés) et un remplissage progressif de l'espace intergranulaire. Malheureusement cette croissance intergranulaire est généralement limitée aux particules situées à proximité immédiate des parois de la cavité osseuse, les autres particules s'entourant de tissus fibreux et donnant ainsi une masse solide. Ainsi, l'ostéoconduction conduit à des particules enrobées progressivement par du tissu osseux qui prolifère à partir des parois de la cavité osseuse, les particules ne réagissant au mieux qu'en surface.

Par ailleurs, quand lesdites particules sont mises en place dans une cavité, par exemple un site d'extraction, elles peuvent migrer dans les tissus mous adjacents, et se trouver fixées dans des endroits impropres. Ceci peut entraîner des complications telles que dysaesthésie ou paraesthésie du nerf..., douleur, infection, perforation de la gencive et perte de particules.

Pour ces raisons, le développement de l'emploi de particules, telles que celles d'hydroxylapatite, passe par l'amélioration de leurs propriétés de mise et de tenue en place. Ceci peut être obtenu par l'emploi d'artifices tels que mise en place à l'aide d'enveloppes résorbables, colles, collagène ou ses dérivés, fibrines adhésives etc...

Ainsi, le remplissage de la cavité devient, après ostéoconduction, un composite de tissus osseux, fibreux et d'apatite ou de bioverre qui a des propriétés mécaniques insuffisantes, inférieures à celles de l'os. Par exemple, l'insertion d'implants prothétiques dans un tel composite ne peut pas être en général pratiqué car elle risque de présenter des défauts de compatibilité, de solidité ou de rigidité dans le temps. De plus, la présence de particules dans les tissus voisins n'est pas souhaitable.

La demande EP 0206726 est ainsi relative à la réparation de défauts parodontaux (poches situées entre l'os de la mâchoire et la racine des dents) par mise en place d'un granulé bioactif dans ledit défaut et illustre l'obtention d'un tel composite os-granulé par ostéoconduction, c'est-à-dire croissance intergranulaire du tissu osseux à partir des parois osseuses du défaut (p.15, 1.13-15 et 25-28). Ledit granulé présente par ailleurs des caractéristiques propres (nature chimique, sélection d'un empilement granulométrique) qui permettent d'obtenir certains avantages, représentant l'objectif principal recherché, comme une bonne cohésion facilitant sa mise et son maintien en place dans le défaut, de même qu'un bouchage permettant d'arrêter l'écoulement sanguin se produisant également dans le défaut. Ce granulé particulier est obtenu par un procédé consistant en une fusion d'un mélange de poudres suivie d'une coulée dans de l'eau sous forme de frittes qui sont lavées à l'acétone puis broyées avant que la sélection granulométrique soit effectuée.

Le granulé décrit dans ce document joue en quelque sorte le rôle d'un implant permanent, objet d'une ostéoconduction, donc s'enrobant progressivement de tissus osseux qui adhère audit implant et le maintient solidement en place; la réparation d'un tel défaut parodontal, conduisant audit type de composite os-verre, ne nécessite pas d'ailleurs des exigences particulières de caractéristiques mécaniques car elle n'est pas en général destinée à recevoir des implantation ultérieures de prothèses.

Le document EP-A-145.210 décrit un autre type de bioverre granulé dont les particules sphéroïdales contiennent SiO₂ ,Na₂O ,P₂O₅ CaO, (par exemple dans les proportions respectives en poids : 45 24,5 6 24,5) et sont obtenues par pulvérisation d'une solution contenant les composés précurseurs, séchage et calcination.

Le document FR-A-2.606.282 décrit une composition pour comblement de cavité osseuse contenant deux composants constitués d'une poudre d'alumine ou d'apatite de granulométrie comprise entre 250 et 1500 µm et d'un liant hydraulique ou organique.

Quant au document EP-A-382.047, il décrit une céramique osseuse résorbable à base de phosphate tricalcique, remplacée progressivement par du tissu osseux et qui est obtenue à partir d'os naturel et d'un composé porteur de phosphate.

Pour pallier les inconvénients résultant d'une ostéoconduction à partir des parois osseuses d'une cavité dans un espace intergranulaire et conduisant auxdits composites os-granulé, la demanderesse a recherché à combler des défauts osseux pratiquement pardu tissu osseux et à accroître la rapidité de formation dudit tissu osseux. Elle a ainsi recherché à produire une ostéostimulation, définie comme étant une ostéogénèse dans un site osseux sans contact avec l'os avoisinant, l'ostéogénèse étant le phénomène conduisant à du tissu osseux, un autre objectif étant d'améliorer les caractéristiques mécaniques du tissu osseux comblant ledit défaut de façon à pouvoir y faire ultérieurement des implantations réussies de prothèse.

L'invention consiste à utilisation de granulés constitués de particules ayant, en combinaison, la composition suivante (en % poids):-
SiO₂ compris entre 40 et 55%;
Na₂O compris entre 10 et 32%;
P₂O₅ compris entre 0 et 12%; et
CaO compris entre 10 et 32%
caractérisée en ce qu'au moins 90% des particules ont une coupe granulométrique comprise entre 300 et 360 *µ*m, comme déterminée par tamisage, avec des arêtes vives, un profil irrégulier et des microdéfauts de surfaces ou microfissures, pour formuler un matériau bioréactif adapté à combler un défaut osseux, les granulés provoquant une ostéogenèse par ostéostimulation, les particules constituantes étant désintégrées de l'intérieur pour opérer une différenciation des cellules précurseurs issues des ostéoblastes, puis être dissoutes et remplacées rapidement par du tissu osseux.

Les cellules précurseurs sont des cellules mesenchymales, présentes à l'origine dans le milieu biologique, qui peuvent se différencier en cellules fibroblastes et chondroblastes qui déposent des tissus fibreux ou cartilagineux (à éviter dans la présente invention) et en cellules ostéoblastes qui déposent du tissu osseux (recherchées dans la présente invention).

Il est important de contrôler la désintégration et la dissolution du granulé car ces phénomènes, s'ils sont incontrôlés, pourraient par exemple donner lieu à une inflammation trop intense qui empêcherait l'apparition des conditions favorables à la différenciation des cellules mesenchymales en ostéoblastes. En outre une dissolution trop rapide des granulés par rapport à la vitesse de formation des tissus osseux ne pourrait plus conduire à une formation de cesdits tissus osseux mais plutôt à la formation de tissus fibreux non désirables.

Les particules constitutives sont ainsi désintégrées de l'intérieur, disparaissent et sont remplacées progressivement et rapidement par du tissu osseux plutôt que par des tissus fibreux; après disparition du matériau, on obtient une masse osseuse se prêtant par exemple parfaitement aux implantations ultérieures de prothèses.
Ce granulé est également d'utilisation et d'application facile; ses particules restant bien en place dans le défaut, ceci évite l'emploi d'artifices.

Il est important que les verres bioréactifs utilisés ne contiennent pas de produits fluorés. Ils contiennent généralement SiO₂, Na₂O, P₂O₅, un oxyde alcalino-terreux, comme CaO. La présence de bore et d'alumine n'est pas souhaitable.

La présence de produits fluorés tel que CaF₂ est néfaste car elle freine trop la restauration et, avec certaines granulométries, peut entraîner par ailleurs une réaction inflammatoire trop intense.

La composition du verre est de préférence comprise dans les fourchettes suivantes:

| | |
|---|---|
| SiO₂ | 42 - 48% |
| Na₂O | 14 - 28% |
| P₂O₅ | 0 - 10% |
| CaO | 20 - 29%. |

Le défaut osseux est de préférence formé dans la mâchoire.

Le matériau doit être sous forme de poudre constituée de particules anguleuses, ayant des arêtes vives et un profil irrégulier. Les particules constitutives doivent présenter des microdéfauts de surface ou des microfissures. Ces microdéfauts ou microfissures seront l'amorce de petits conduits qui, comme on le verra plus loin, relient l'intérieur des particules à l'extérieur et favorisent leur désintégration par l'intérieur.

Ces caractéristiques de taille et de morphologie sont essentielles pour pouvoir utiliser les bioverres décrits ci-dessus avec les résultats recherchés.

En général, les particules selon l'invention ont été implantées dans des chiens, puis des échantillons ont été prélevés après 1, 2, 3, 6 mois, un an; après traitement de fixation, ils ont été découpés au microtome et préparés pour être observés par microscopie et analysés.

Avec un matériau selon l'invention, la demanderesse a constaté que, non seulement le tissu osseux se développe à partir de la paroi de la cavité osseuse, par ostéoconduction, en formant un squelette osseux, mais encore de façon inattendue, que les particules situées au milieu de la cavité, donc sans contact avec les parois, se désintègrent et s'ossifient simultanément à partir de leur centre, cette désintégration ne produisant pas de réaction inflammatoire chronique nocive.

A l'intérieur desdites particules, il se forme une excavation par désintégration et dissolution de la partie centrale sous l'action du liquide physiologique et de cellules macrophages, histiocytes, plasmocytes et lymphocytes. Ces excavations centrales sont reliées à l'extérieur par de petits conduits. Ensuite on note que, sur la paroi de l'excavation formée, s'étend du tissu osseux recouvert par une couche de tissu ostéoide et une bande de cellules actives ostéoblastes. On remarque ainsi qu'il s'est opéré la différentiation souhaitée mais inattendue des cellules mesenchymales en ostéoblastes plutôt qu'en fibroblastes. Un bon nombre de ces excavations est déjà pratiquement rempli de tissu osseux au boût de 3 mois. Il n'y a pas de liaison directe entre le tissu osseux se développant au centre des particules et le tissu osseux extérieur.

Une explication peut être que les particules implantées sont exposées à l'action du liquide physiologique et de cellules; ces derniers opèrent une dissolution et pénètrent à l'intérieur desdites particules par l'intermédiaire de défauts de surface ou de microfissures, dus au procédé de fabrication du granulé et accessibles audit liquide; il se produit des changements locaux de pH et de composition du verre, qui sont favorables à l'ostéostimulation, celle-ci favorisant la transformation préférentielle des cellules mesenchymales précurseurs en ostéoblastes déposant du tissu osseux; il n'y a pas, en particulier, de développement des cellules fibroblastes, générant des tissus fibreux, gênants, au contact du verre ayant réagi. L'intérieur des particules constitue un milieu très protégé qui permet de créer et de maintenir lesdites conditions faborables à la différenciation des cellules mesenchymales en ostéoblastes. Cela signifie que le granulé de remplissage est ostéostimulant dans les conditions spécifiques et limitées de l'invention. En particulier les particules trop petites, donnent une réaction trop intense et conduisent à du tissus fibreux, ces conditions étant défavorables à la différenciation des cellules mesenchymales en ostéoblastes.

En fin d'évolution on obtient ainsi, dans le défaut ossseux, une matière de remplissage également osseuse, organiquement liée à la paroi dudit défaut; ce remplissage a donc les propriétés de l'os et peut être utilisé dans les mêmes conditions que l'os voisin.
De plus, on ne constate pas de migration de particules dans les tissus non osseux avoisinant ledit défaut osseux.

Le matériau selon l'invention n'est pas stable, il est au contraire actif, il est stimulateur et entraîne de façon inattendue la formation du tissu osseux par ostéostimulation. Il permet donc d'obtenir une ossification beaucoup plus rapide puisqu'elle se fait non seulement à partir de la paroi de la cavité en enrobant les particules, mais également et simultanément dans la masse par l'intérieur des particules.

Les caractéristiques de taille et de morphologie sont essentielles pour pouvoir obtenir les résultats recherchés.
En effet, quand on met les particules en contact avec le mi lieu physiologique, il se constitue une couche superficielle riche en phosphate de calcium (CaP) tandis qu'au contraire la couche sous-jacente devient riche en SiO₂.
Si les particules sont de trop petites dimensions, leur intérieur est constitué d'un gel de silice recouvert d'une couche de CaP trop mince et très fragile; celle-ci a tendance à se briser, les cellules macrophages absorbent la silice et les particules disparaissent trop rapidement sans que le milieu, très protégé et faborable à la différenciation des cellules précurseurs en ostéoblaste, constitué par l'excavation intérieure de la particule, ait eu le temps de se former et de donner naissance à du tissu osseux. Avec ce type de particule, il n'y a pas d'ostéostimulation.
De même si dans une coupe granulométrique lesdites particules de trop petites dimensions sont en quantité trop importante, la réaction trop intense qu'elles provoquent peut entraîner une inhibition de l'ostéostimulation qui pourrait se produire avec des particules de taille plus élevée.

Si les particules ont une granulométrie conforme à l'invention, la couche de CaP est plus solide et le centre contient de la silice mais pas de verre. Le CaP ne se brisant plus, les cellules macrophages peuvent pénétrer dans la particule par les microdéfauts et créer l'excavation; le milieu très protégé et favorable à la différenciation peut ensuite se développer; il y a alors ostéogénèse par ostéostimulation.

Si les particules sont de trop grandes dimensions, il reste du verre à l'intérieur desdites particules, on ne note aucune formation d'excavation, et par conséquent la différenciation souhaitée ne se produit pas; les grosses particules subsistent.

A la différence de l'art antérieur, on voit bien que la composition, la distribution granulométrique et la morphologie des particules sont des facteurs essentiels pour que l'on observe cette ostéostimulation par dissolution et désagrégation desdites particules de leur intérieur, leur disparition et leur remplacement par du tissu osseux.

Etant donné que le but est de combler le défaut osseux aussi rapidement que possible, il est essentiel centre contient de la silice mais pas de verre. Le CaP ne se brisant plus, les cellules macrophages peuvent pénétrer dans la particule par les microdétauts et créer l'excavation; le milieu très protégé et favorable à la différenciation peut ensuite se développer; il y a alors ostéogénèse par ostéostimulation.

Si les particules sont de trop grandes dimensions, il reste du verre à l'intérieur desdites particules, on ne note aucune formation d'excavation, et par conséquent la différenciation souhaitée ne se produit pas; les grosses particules subsistent.

A la différence de l'art antérieur, on voit bien que la composition, la distribution granulométrique et la morphologie des particules sont des facteurs essentiels pour que l'on observe cette ostéostimulation par dissolution et désagrégation desdites particules de leur intérieur, leur disparition et leur remplacement par du tissu osseux.

Etant donné que le but est de combler le défaut osseux aussi rapidement que possible, il est essentiel d'avoir un granulé entièrement dans la coupe granulométrique revendiquée. Les granulés en dehors de cette coupe ne produisent pas d'ostéostimulation et, par conséquent, l'effet recherché qui conduit à une formation osseuse précoce n'existe pas.

On voit que l'invention produit une dissolution préférentielle du verre en opposition aux mécanismes de réaction des bioverres de composition comparable décrits dans l'art antérieur. En effet lesdits mécanismes décrits sont en général des réactions de lessivage de certains ions à la surface du verre, sans qu'il ne soit noté une dissolution dudit verre.

Par ailleurs, l'état de surface rugueux des granulés, dû à leurs caractéristiques de forme, agit de façon agglutinante ou imbriquante et est tel que le matériau est de mise en place facile, les particules restant en place dans la cavité en s'agglutinant entre elles et en s'agrippant aux parois de la cavité osseuse. Bien entendu, pour des applications particulières, il est possible d'utiliser d'autres artifices de mise en place.

D'une façon habituelle on obtient le matériau selon l'invention en préparant tout d'abord un mélange de poudres comprenant de la silice, un oxyde alcalino terreux (de préférence CaO et/ou MgO), un carbonate pour introduire l'alcalin au moins en partie (on utilise de préférence le carbonate de sodium), un phosphate ou phosphate acide pour introduire P₂O₅ (de préférence CaHPO₄).

On fond ce mélange et on le coule sous forme de pièces, habituellement de petits cylindres ou disques, dans des moules relativement massifs en matériau bon conducteur de la chaleur (par exemple graphite, métal...). Un recuit peut être effectué. Ces pièces sont ensuite réduites en granulé par tous moyens permettant de donner des particules fissurées, rugueuses ayant de très nombreux angles vifs. On emploie de préférence le concassage et/ou le broyage, par exemple en mortier, à marteau, à barre. Puis on réalise la coupe granulométrique souhaitée par exemple par passage sur tamis vibrants.

Il est important qu'avant broyage les pièces coulées ne soient pas en contact avec un liquide (par exemple eau, acétone...) ou tout autre élément pouvant réagir avec le verre. En effet, il pourrait alors se produire des modifications (chimiques et/ou physique) telles que les microdéfauts ou microfissures n'apparaissent plus.

### EXEMPLES

Pour illustrer l'invention, on a comparé un matériau selon l'invention à d'autres matériaux ne répondant pas aux conditions.

### Exemple 1

On est parti d'un mélange de poudres contenant (% poids) :

| | |
|---|---|
| SiO₂ | 45 % |
| CaO | 24.5 % |
| Na₂O | 24,5 % |
| P₂O₅ | 6 % |

Ce mélange a été fondu et coulé sous forme de petits disques (diamètre 4 cm. hauteur 1 cm) qui ont une ligne transversale et indicatives pour les teneurs en CaP et en gel de silice.

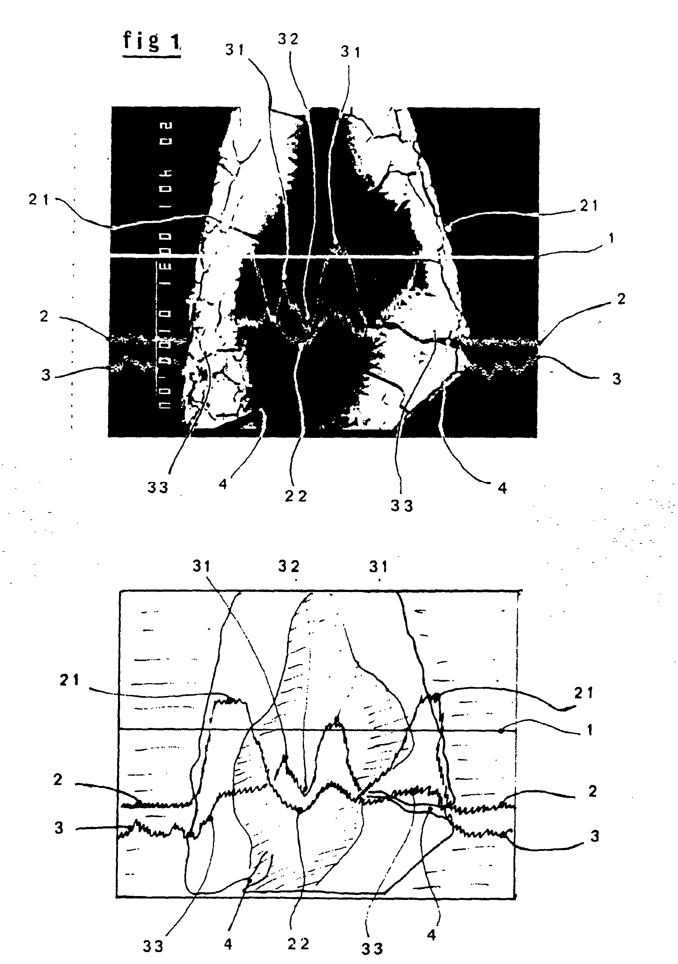

La figure 1 représente une particule selon l'invention (grossissement X 400) après un mois d'implantation. On observe, en clair, la couche extérieure riche en CaP et, en sombre, l'excavation centrale en partie désintégrée. Les analyses ont été effectuées selon la ligne transversale (1); le long de cette ligne, la teneur en Ca est donnée par la courbe (2) et celle en Si par la courbe (3).
On voit en (21) la couche extérieure riche en CaP, alors qu'au centre de la particule la teneur en CaP est pauvre (22). En ce qui concerne SiO₂ on voit plusieurs zones : une zone pauvre à la fois en SiO₂ (32), en cours de désintégration, et en CaP (22), deux zones (33) pauvres en SiO₂ à l'extérieur desquelles la couche de CaP (21) est en train de se former, deux zones (31) contenant de la silice non encore dissoute. En (4) on voit les conduits reliant l'excavation à l'extérieur par l'intermédiaire, au départ, de microdéfauts de surface.

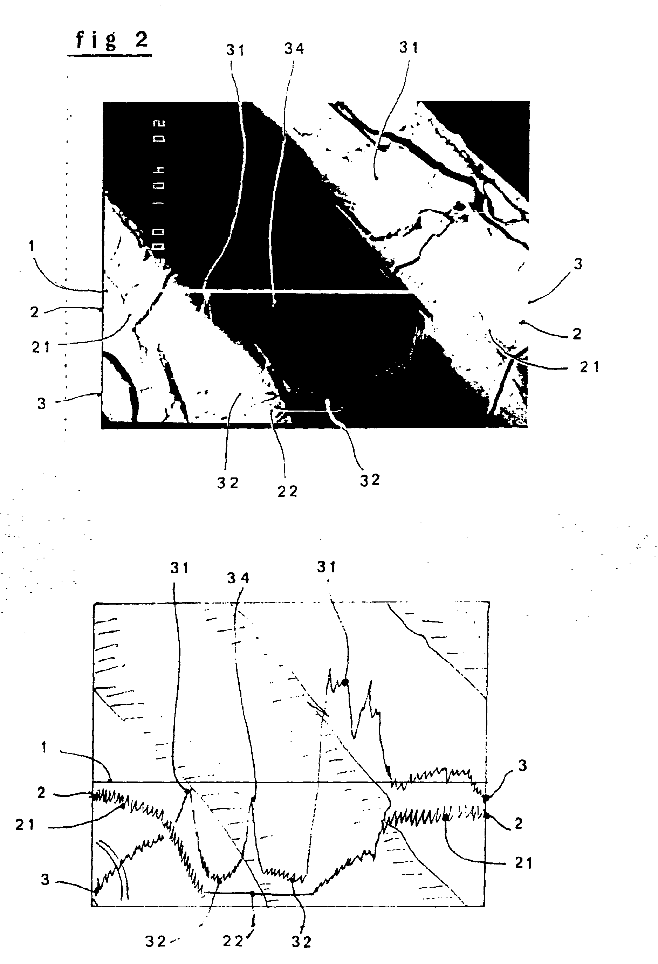

La fig. 2 représente également une particule selon l'invention après 3 mois d'implantation. Alors que dans la fig. 1 l'excavation est en cours de formation, dans la fig. 2 cette excavation est formée. Les repères ont la même signification que dans la fig. 1.
On note cependant un pic (34) de concentration en Si qui n'a pas de signification particulière.

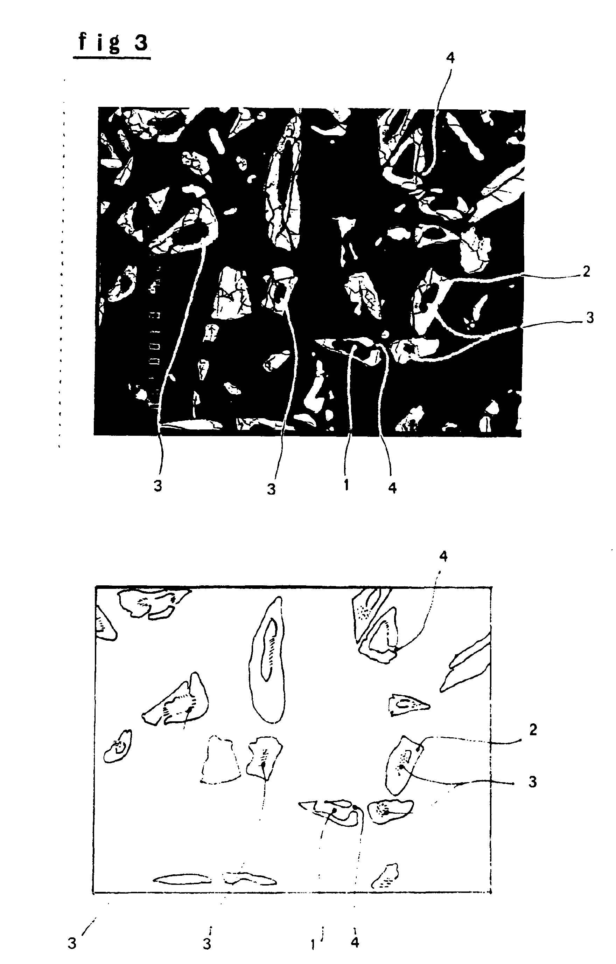

La figure 3 représente un ensemble de particules (grossissement x 60) après 3 mois. On voit, en sombre, les excavations intérieures (1) à chaque particule entourées de la couche claire (2) riche en CaP. La présence de tissu osseux est visible en (3) en plus ou moins grande quantité.
On voit en (4) les conduits de communication de l'excavation avec l'extérieur. Comme cela a été indiqué précédemment, on ne note pas la présence de tissu osseux dans le milieu extérieur.
La coupe, étant bidimensionnelle, peut avoir été effectuée près du sommet de certaines particules, ce qui explique la présence sur la fig. 3 de particules apparemment petites et sans excavation.

### Exemple 2

Cet exemple montre les résultats imparfaits obtenus avec un granulé comportant un excès de grosses particules (supérieures à 500 µm).
Les conditions d'expérimentation sont identiques à celles de l'exemple 1; seulement la coupe granulométrique a été choisie plus grosse en utilisant des tamis de 425 à 850 µm.
Dans ce cas, on note toujours une croissance externe du tissu osseux (ostéoconduction), à partir de la paroi de la cavité osseuse, entre les particules et le long de leurs surfaces. Au centre de la cavité, lesdites particules sont entourées de tissus fibreux, consistant en fibres collagènes entassées de façon dense. Les grosses particules ne montrent pas de désintégration centrale avec résorbtion ou dissolution de verre et substitution par du tissu osseux. Il n'est pas évident qu'une ossification des grosses particules se produise après des durées d'implantation beaucoup plus longues, car elles peuvent se trouver enrobées et enfermées dans du tissus osseux formé par ostéoconduction, ce tissu empêchant toute réaction accrue telle qu'elle existait au début de l'implantation.

### Exemple 3

Il illustre les résultats imparfaits de restauration dûs à l'emploi de granulés trop fins.
Les conditions d'expérimentation sont identiques à celles de l'exemple 1; seule la coupe granulométrique a été choisie plus petite, en utilisant des tamis de 212 et 300 µm.
On note également dans ce cas une croissance externe du tissu osseux par ostéoconduction à partir de la paroi de la cavité et entre les particules, lequel tissu enrobe et se soude auxdites particules. Au centre de la cavité osseuse, la différenciation des cellules précurseurs en ostéoblastes ne s'est pas faite et les particules sont entourées de tissus fibreux avant de disparaître sous l'action de cellules phagocytantes.
Seule quelques grosses particules montrent une désintégration centrale avec substitution de leur coeur par les cellules décrites dans l'exemple 1, et par du tissu osseux.

### Exemple 4

Il illustre les mauvais résultats obtenus avec un granulé contenant un fluorure. Le verre a la composition suivante (% poids) :

| | |
|---|---|
| SiO₂ | 52 % |
| CaO | 16 % |
| Na₂O | 10 % |
| P₂O₅ | 6 % |
| CaF₂ | 16 % |

et on a utilisé une granulométrie 425-800 *µ*m.
Après implantation de 3 mois on note, malgré une grosse granulométrie, une reaction inflammatoire trop intense, phénomène nocif qui n'a pas été observe avec le granule de l'invention. Cette reaction inflammatoire est attributable à la presence de fluorure.

On voit que le granule selon l'invention favorise la reaction tissulaire d'ostéogénèse par ostéostimulation qui se product en tous points du défaut osseus (y comprise la zone centrale) à l'intérieur des particles en les détruisant et en les remplaçant par du tissu osseus, sans empêcher par ailleurs une ostéoconduction qui tend à enrober les particles, sans les détruire, à parti des parois de la cavité osseuse.

## Revendications

1. Utilisation de granulés constitués de particules ayant, en combinaison, la composition suivante (en % poids):-
SiO₂ compris entre 40 et 55%;
Na₂O compris entre 10 et 32%;
P₂O₅ compris entre 0 et 12%; et
CaO compris entre 10 et 32%
**caractérisée en ce qu'**au moins 90% des particules ont une coupe granulométrique comprise entre 300 et 360 *µ*m, comme déterminée par tamisage, avec des arêtes vives, un profil irrégulier et des microdéfauts de surfaces ou microfissures, pour formuler un matériau bioréactif adapté à combler un défaut osseux, les granulés provoquant une ostéogenèse par ostéostimulation, les particules constituantes étant désintégrées de l'intérieur pour opérer une différenciation des cellules précurseurs issues des ostéoblastes, puis être dissoutes et remplacées rapidement par du tissu osseux.

2. Utilisation selon la revendication 1, **caractérisée en ce que** la composition du verre bioréactif est la suivante (en % poids):
SiO₂ compris entre 42 et 48%;
Na₂O compris entre 14 et 28%;
P₂O₅ compris entre 0 et 10%; et
CaO compris entre 20 et 29%.

3. Utilisation selon l'une quelconque des revendications 1 à 2, dans laquelle le défaut osseux est formé dans la mâchoire.

## Patentansprüche

1. Verwendung von Körnchen, die aus Teilchen bestehen, die in Kombination die folgende Zusammensetzung haben (in Gew.%):
SiO₂ im Bereich von:40 bis 55%
Na₂O im Bereich von 10 bis 32%
P₂O₅ im Bereich von 0 bis 12%
CaO im Bereich von 10 bis 32%,
**dadurch gekennzeichnet, dass** wenigstens 90% der Teilchen in einem durch Siebung ermittelten Komgrößenbereich von 300-360 µm liegen, mit scharfen Kanten, einem unregelmäßigen Profil und Mikrooberflächenfehlern oder Mikrorissen, so dass sie ein bioaktives Material bilden, das dazu geeignet ist, eine Knochenhöhle zu füllen, indem die Kömchen eine Knochenbildung durch Knochenstimulierung hervorrufen, wobei die Teilchen von Innen desintegriert werden, um eine Differenzierung von Vorläuferzellen in Osteoblaste zu bewirken und dann aufgelöst und in kurzer Zeit durch Knochengewebe ersetzt zu weiden.

2. Verwendung nach dem Anspruch 1, **dadurch gekennzeichnet, dass** die Zusammensetzung des bioreaktiven Glases die folgende (in Gew.%) ist:
SiO₂ im Bereich von :42 bis 48%
Na₂O im Bereich von 14 bis 28%
P₂O₅ im Bereich von: 0 bis 10%
CaO im Bereich von :20 bis 29%.

3. Verwendung nach irgendeinem der Ansprüche 1 bis 2, wobei die Knochenhöhle im Kiefer gebildet ist.

## Claims

1. The use of granules constituted by particles having in combination the following composition (% by weight):-
SiO₂ between 40 and 55%;
NA₂O between 10 and 32%;
P₂O₅ between 0 and 12%; and
CaO between 10 and 32%
**characterized in that** at least 90% of the particles have a grain size fraction between 300 and 360 *µ*m as determined by screening, with sharp edges, an irregular profile and surface microdefects or microcracks to formulate a bioactive material adapted to fill an osseous cavity, the granules bringing about an osteogenesis by osteostimulation, the constituent particles being disintegrated from the inside in order to bring about a differentiation of the precursor cells from osteo-blasts followed by dissolving and rapid replacement by osseous tissue.

2. The use according to claim 1, **characterized in that** the composition of the bioreactive glass is as follows (in % by weight):
SiO₂ between 42 and 48%;
NA₂O between 14 and 28;
P₂O₅ between 0 and 10%; and
CaO between 20 and 29%

3. The use according to any one of claims 1 to 2 wherein the osseous cavity is formed in the jaw.
